# EUROPEAN PATENT SPECIFICATION

(11) **EP 1 313 876 B1**
(45) Date of publication and mention of the grant of the patent: **03.01.2007**
(21) Application number: 01974181.8
(22) Date of filing: 13.08.2001
(51) Int. Cl.: C12Q 1/42, G01N 33/573, C12Q 1/34, C12Q 1/48

(54) **PROCESS FOR DETECTING ACETYLASE OR DEACETYLASE ACTIVITY IN AN IMMUNOASSAY**
VERFAHREN ZUM NACHWEIS VON ACETYLASE ODER DEACETYLASE AKTIVITÄT IN EINEM IMMUNOASSAY
PROCEDE DE DETECTION D'ACTIVITE D'UN ACETYLASE OU DEACETYLASE DANS UN DOSAGE IMMUNOLOGIQUE

(30) Priority: 11.08.2000 EP 00117457; 24.08.2000 EP 00118431; 18.10.2000 EP 00122707; 21.12.2000 EP 00128176
(43) Date of publication of application: 28.05.2003
(73) Proprietor: Evotec OAI AG, 22525 Hamburg (DE)
(72) Inventor: KRÄMER, Joachim, 25474 Ellerbek (DE); PEIKER, Christine, 25469 Halstenbek (DE); HENCO, Karsten, 40699 Erkrath (DE)
(74) Representative: Hertz, Oliver
(86) International application number: PCT/EP2001/009354
(87) International publication number: WO 2002/014543

(56) References cited:
- WO-A-99/29894
- KHOKHLATCHEV ANDREI ET AL: "Reconstitution of mitogen-activated protein kinase phosphorylation cascades in bacteria: Efficient synthesis of active protein kinase" JOURNAL OF BIOLOGICAL CHEMISTRY, AMERICAN SOCIETY OF BIOLOGICAL CHEMISTS, BALTIMORE, MD, US, vol. 272, no. 17, 1997, pages 11057-11062, XP002163437 ISSN: 0021-9258
- BORGNE A & MEIJER L: "Sequential dephosphorylation of p34cdc2 on Thr-14 and Tyr-15 at the prohase/metaphase transition" JOURNAL OF BIOLOGICAL CHEMISTRY, AMERICAN SOCIETY OF BIOLOGICAL CHEMISTS, BALTIMORE, MD, US, vol. 271, no. 44, 1996, pages 27847-27854, XP002162637 ISSN: 0021-9258
- CLAYTON ALISON L ET AL: "Phosphoacetylation of histone H3 on c-fos- and c-jun-associated nucleosomes upon gene activation." EMBO (EUROPEAN MOLECULAR BIOLOGY ORGANIZATION) JOURNAL, vol. 19, no. 14, 17 July 2000 (2000-07-17), pages 3714-3726, XP002225679 ISSN: 0261-4189
- SEETHALA RAMAKRISHNA ET AL: "A fluorescence polarization competition immunoassay for tyrosine kinases" ANALYTICAL BIOCHEMISTRY, ACADEMIC PRESS, SAN DIEGO, CA, US, vol. 255, 15 January 1998 (1998-01-15), pages 257-262, XP002150326 ISSN: 0003-2697
- WU J J ET AL: "IDENTIFICATION OF A HIGH-AFFINITY ANTI-PHOSPHOSERINE ANTIBODY FOR THE DEVELOPMENT OF A HOMOGENEOUS FLOURESCENCE POLARIZATION ASSAY OF PROTEIN KINASE C" JOURNAL OF BIOMOLECULAR SCREENING, LARCHMONT, NY, US, vol. 5, no. 1, February 2000 (2000-02), pages 23-30, XP000992190 ISSN: 1087-0571
- CHEN TING ET AL: "Interaction of phosphorylated Fc-epsilon-RI-gamma immunoglobulin receptor tyrosine activation motif-based peptides with dual and single SH2 domains of p72-syk. Assessment of binding parameters and real time binding kinetics." JOURNAL OF BIOLOGICAL CHEMISTRY, vol. 271, no. 41, 1996, pages 25308-25315, XP002225680 ISSN: 0021-9258
- DATABASE BIOSIS [Online] BIOSCIENCES INFORMATION SERVICE, PHILADELPHIA, PA, US; August 1999 (1999-08) BARANCIK MIROSLAV ET AL: "Okadaic acid and anisomycin are protective and stimulate the SAPK/JNK pathway." Database accession no. PREV199900492152 XP002225692 & JOURNAL OF CARDIOVASCULAR PHARMACOLOGY, vol. 34, no. 2, August 1999 (1999-08), pages 182-190, ISSN: 0160-2446

## Description

The present invention relates to a process for detecting acetyltransferase or deacetylase activity in an immunoassay.

In eukaryotic cells there are numerous post-translational modifications of proteins which are introduced after termination of the protein synthesis. In the post-translational modification of proteins the action of corresponding modification enzymes is required. There are a great number of post-translational modifications known which are introduced after termination of the protein synthesis.

As an example, particular chemical groups may be added to amino acids in a protein. Representative examples are the glycosylation, phosphorylation, acetylation, acylation and carboxylation. Also, particular chemical groups may be removed from a protein by processes like e.g. deglycosylation, dephosphorylation, deacetylation, deacylation and decarboxylation.

An example of the importance of enzymatic modifications shall be given with view to acetyltransferases and deacetylases. The chromatin structure has an influence on key cellular processes such as DNA replication, transcription, DNA repair and differentiation. The chromatin structure and the binding of regulatory_proteins to DNA can be modified by reversible acetylation of the tertiary amino groups of conserved lysine residues in the N-terminal tails of core histones. This enzymatic modification is controlled by histone acetyltransferases and histone deacetylases. Increasing evidence is accumulating that inhibitors of histone deacetylase may have a great potential in cancer therapy (Nakayama J. et al., Science, Vol. 292, 110 - 113, 2001; Darkin-Rattray et al., Proc. Natl. Acad. Sci. USA, Vol. 93, 13142 - 13147, 1996; Kölle et al., Methods: A companion to methods in enzymology, Vol. 15, 323 - 331, 1998). Inhibitors of histone deacetylase are of great potential as new drugs due to their ability to influence transcriptional regulation and to induce apoptosis or differentiation in cancer cells.

So far no assay formats exist to measure the activity of histone deacetylases and potential modulators (i.e. inhibitors or activators) of said enzyme activity which are compatible with the requirements of high throughout screening, such as homogeneous (i.e. "mix, incubate and read") assay format, high sensitivity and short measurement time.

A. L. Clayton et al. (The EMBO Journal, vol. 19, 2000, pp. 3714-3726) describe the phosphoacetylation of histone H3 on *c-fos-* and *c-jun*-associated nucleosomes upon gene activation. The application of high throughput screening procedures with fluorescence measurement is described in particular by R. Seethala et al. (Analytical Biochemistry, vol. 255, 1998, pp. 257-262) and J.J. Wu (Journal of Biomolecular Screening, vol. 5, 2000, pp. 23-30) as well as in WO 99/29894.

From the foregoing, it will be clear that there is a continuing need for the development of cost-effective, facile and sensitive enzymatic assays for both high throughput screening (HTS) of potential drugs and secondary assays.

It was therefore a main object of the present invention to establish an assay method for detecting the activity of acetyltransferases and deacetylases. The assay methods should be highly reliable and simple to perform.

The above mentioned main object has been solved by the assay process according to the features of claim 1.

In particular, the present invention relates to a process for detecting acetylation or deacetylation of a substrate by virtue of acetylase or deacetylase enzyme activity, respectively, in an immunoassay which comprises the following steps:
a) providing a protein, a peptide, or a derivative thereof comprising the sequence motif

   -Z-X-Y- or -Y-X-Z_

   wherein
   Z = an amino acid to be acetylated or deacetylated by the respective enzyme,
   X = a sequence of amino acids, preferably between 0 and 10 amino acids which may be the same or different,
   Y = a discrimination enhancer for binding to an antibody, which discrimination enhancer comprises a phosphorylated amino acid
   as a substrate for the enzyme;
b) incubating the protein, peptide, or derivative thereof with the enzyme to form a protein, peptide, or derivative thereof which is acetylated or deacetylated, respectively, at the Z position;
c) adding an antibody discriminating the modified Z position from the unmodified Z position of said protein, peptide, or derivative thereof, said discrimination being mediated by the presence of the enhancer; and
d) detecting the enzyme activity applying a homogeneous fluorescence binding immunoassay.

In the assay for detecting acetylase / deacetylase activity, the discrimination enhancer comprises a phosphorylated amino acid.

Sometimes, the terms "discrimination enhancer" or "enhancer" will be used as synonymous to the term "affinity enhancer".

The further subclaims define preferred embodiments of the process of the present invention.

In one embodiment, the substrate might be combined with said enzyme before the addition of said antibody. However, all assay components might also be combined simultaneously.

In the sequence motif, any amino acid or sequence of amino acids (X) can be inserted between Z and Y. The number of amino acids is in the range of 0 to 10 amino acids. A range of 0 to 10 is preferred to include both linear and conformational antibody epitopes. X is particularly at least one amino acid, any other short amino acid sequences having at least two amino acids, such as oligopeptides, being also included.

The antibody used is usually a monoclonal or polyclonal antibody. The antibody to be used discriminates the modified Z position from the unmodified Z position of the substrate.

According to the present invention, this discriminating capability is mediated by the presence of a discriminating enhancer in the substrate's amino acid sequence. The discriminating enhancer Y contained in the sequence motif has the function to promote and/or intensify the binding of an antibody which is added in step (c) of the process of the present invention. The enhancer Y can comprise at least one amino acid which is modified or substituted, by a phosphate,

Depending on the enzyme modification reaction such as the enzymatic addition or elimination of groups to or from the protein, peptide, or derivative thereof, the enzyme may be of such kind that either the addition or the elimination of the chemical group is promoted by the enzyme acetylase or deacetylase

In the event that the protein, peptide, or derivative thereof is enzyme-modified by addition of chemical groups to the Z position in the sequence motif, a corresponding co-substrate is used in step (b). Suitable co-substrates comprise The immunoassay according to the present invention may be performed as a direct immunoassay, preferably a homogeneous direct binding immunoassay.

Any homogeneous assay is based on a mix and measure principle. This is highly advantageous over the conventional' direct binding assays always requiring complicated separation steps of the assay components before detection.

In the direct binding immunoassay, typically either a detectable peptide/protein/derivative thereof, or a detectable antibody is used. Usually, detection takes place by optical methods and any beforehand labelling of the peptide etc. or the antibody may be carried out according to conventional standard techniques. Preferably, the peptide/protein/derivative thereof and/or the antibody are labelled using a luminescent tag or by using specific labelling molecules such as a reporter enzyme or an affinity ligand.

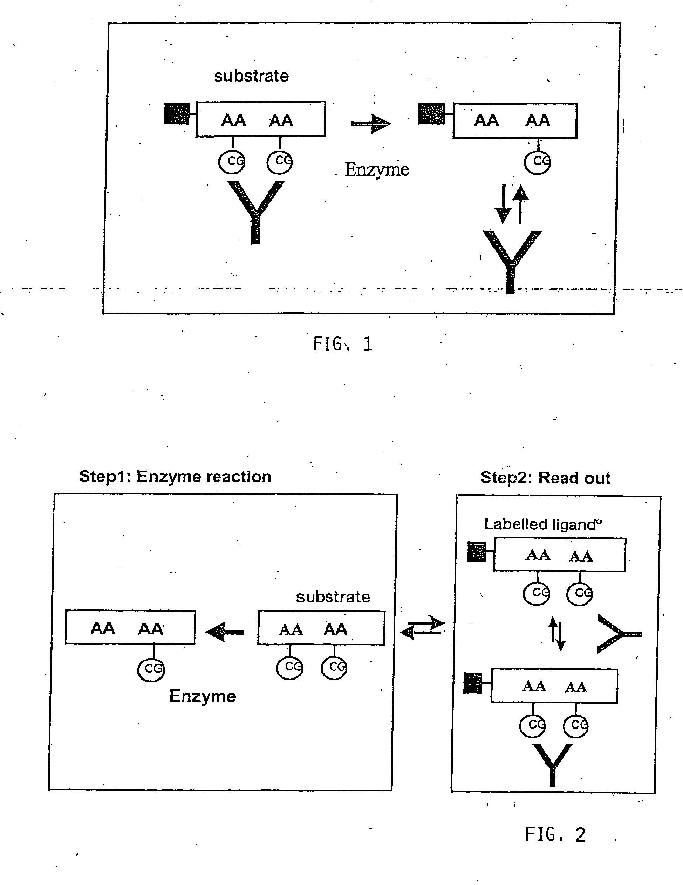

In figure 1 a schematic drawing of one embodiment of the direct assay of the present invention is shown. A labelled modified protein/peptide substrate carries two chemical groups (CG) on two amino acids (AA) in the Z and Y position. By the action of an enzyme, one of the CGs is cleaved off. Consequently, the labelled modified substrate does no longer bind to the specific antibody.

The assay according to the present invention may also be performed as a competition immunoassay, preferably a homogeneous indirect binding assay. Also, a homogeneous indirect binding assay is based on the mix and measure principle.

In this indirect binding assay, a detectable ligand is added to compete with either the modified or the unmodified form of the peptide/protein/derivative for binding to an antibody. The ligand is preferably made optically detectable or labelled using a luminescent tag or by using specific labelling molecules such as a reporter enzyme or an affinity ligand.

Figure 2 shows a schematic drawing of an of the indirect assay of the invention. In a first step, a modified peptide/protein/ substrate having two chemical groups (CG) on two amino acids (AA) in the Z and Y position is subjected to the action of an enzyme, consequently loosing one of the chemical groups. The product of the reaction does no longer compete with the ligand for binding to the specific antibody.

The assay of the present invention can be performed as a fluorescence immunoassay. In particular, fluorescence immunoassays such as a fluorescence polarization (FP) immunoassay, a fluorescence correlation spectroscopy (FCS) assay, a fluorescence lifetime (FL) assay, or a fluorescence intensity distribution analysis (FIDA) assay may be mentioned.

As will also be outlined in more detail below, the invention relates to a process for detecting acetylase or deacetylase activity. Therefore, also a specific kit is used for performing the corresponding assays. Such a kit comprises the following components:
(i) a substrate comprising the sequence motif

   -Z-X-Y- or Y-X-Z-

   wherein
   Z = an amino acid to be acetylated or deacetylated by a respective enzyme,
   X = a sequence of amino acids, preferably between 0 and 10 amino acids which may be the same or different,
   Y = a discrimination enhancer for the binding to an antibody, said discrimination enhancer comprising a phosphorylated amino acid; and
(ii) an antibody discriminating the modified Z from the unmodified Z position of said substrate, said discrimination being mediated by the presence of the discrimination enhancer.

The processes according to the present invention as well as the kits may be used for screening modulators (inhibitors or activators) for enzyme activity. Such modulators may play a key role in metabolism of animals including human beings. They are considered to be extremely useful for the treatment of diseases, such as metabolic disorders and cancer.

The accompanying figures illustrate the present invention.

In the figures the following is shown:
- Figure 1: is a schematic drawing of the principle of the direct assay of the present invention.
- Figure 2: is a schematic drawing of the principle of the indirect assay of the present invention.

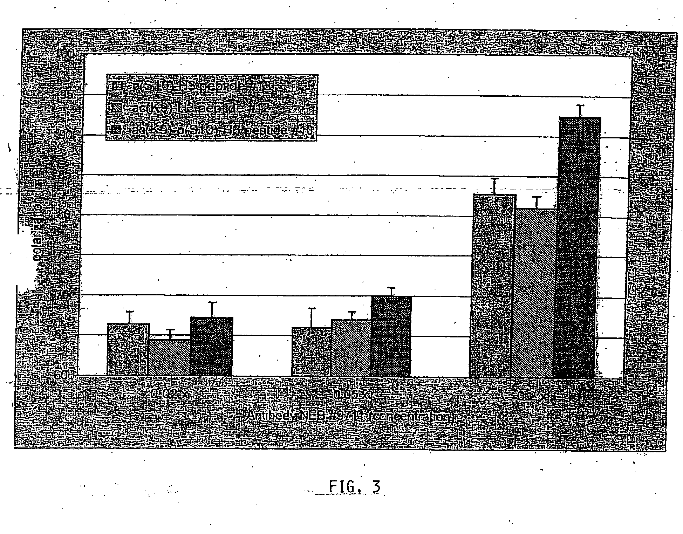

- Figure 3: is a schematic drawing showing the binding of an ac(K9)-p(S10)- specific antibody from New England Biolabs (# 9711) to dual-modified ac(K9)p(s10)-H3 Peptide #10; as a control, only low binding is measured for mono-modified ac(K9)-H3 peptide #12 and p(S10)-H3 Peptide #13.

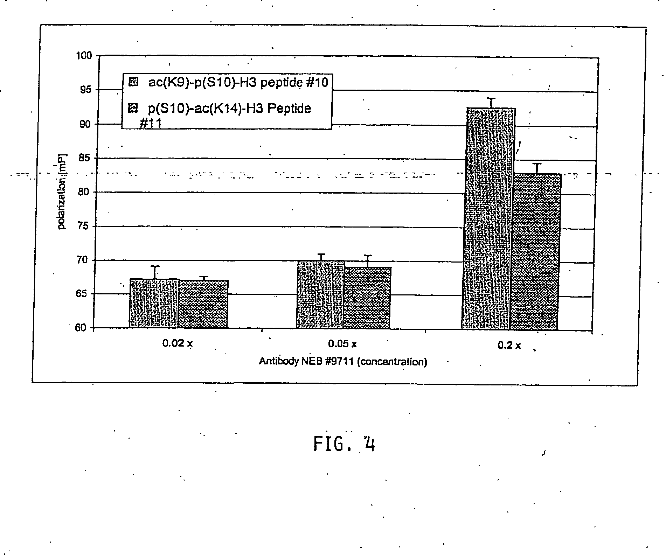

- Figure 4: is a schematic drawing showing the binding of an ac (K9) -p(S10)-specific antibody from New England Biolabs (# 9711) to dual-modified ac(K9)p(S10)-H3 Peptide #10; as a control, only low binding is measured for dual-modified p(S10)ac(K14)-H3 Peptide #11.

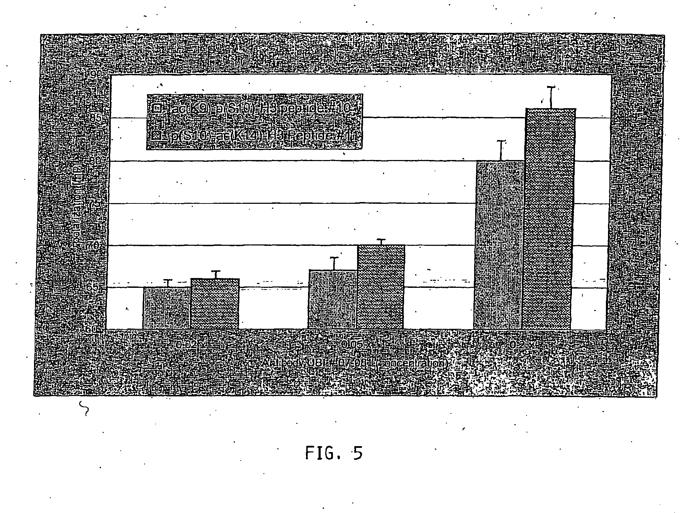

- Figure 5: is a schematic drawing showing the binding of an p(S10)-ac(K14)-specific antibody from Upstate Biotech (# 07-081) to dual-modified p(S10)ac(K14)-H3 peptide #11; as a control, only low binding is measured for dual-modified ac(K9)p(S10)-H3 peptide #10.

The following experiments further illustrate the present invention.

### EXAMPLE 1

### Acetylation of lysines in proteins

The histone deacetylase assay uses fluorescently-labelled peptides (TAMRA dye as a label) derived from histone H3 sequence. In the assay two different polyclonal antibodies are used which bind specifically to acetylated lysine redidues (ac(K) at positions ac(K9) or ac(K10)). Acetylation of a single lysine results in a relative small change of the molecular structure of the aminoacid side chain. The resulting change in the binding affinity to acetyl-specific antibodies is in general difficult to detect in a homogeneous binding assay. According to the invention, therefore an additional modification (phosphorylation of serine at position (S10)) was included within the H3-peptide sequence to enhance the affinity of the peptides to the applied antibodies. From the results of three independing binding experiments (figures 15 - 17) it is evident that dual-modified peptide conjugates confer a higher affinity for the applied antibodies when compared to mono-modified peptide conjugates. This characteristic is of great value to measure the different acetylation states of lysine residues using acetyl-specific antibodies. In principle, the described binding assay can be applied both for the detection of acetyltransferase or deacetylase activity, in particular histone acetyltransferase / deacetylase.

### Reagents and buffers:

### Fluorescent TAMRA-Peptide conjugates:

All fluorescent peptides were supplied by Evotec BioSystems AG. Amino acids are given in the one-letter code. Ac denotes acetylated, p denotes phosphorylated.
ac(K9)p(S10)-H3 peptide #10:
TAMRA-A-R-ac(K9)-p(S10)-T-T-G-K-A-P
p(S10)ac(K14)-H3 peptide #11:
TAMRA-A-R-K-p(S10)-T-T-G-ac(K14)-A-P
ac(K9)-H3 peptide #12:
TAMRA-A-R-ac(K9)-S-T-T-G-K-A
p(S10)-H3 peptide #13:
TAMRA-A-R-K-p(S10)-T-T-G-K-A

### Antibodies:

1. Polyclonal ac (K9)-p (S10) -specific antibody from New England Biolabs, U.S.A., (#9711)
2. Polyclonal p(S10)-ac(K14)-specific antibody from Upstate Biotech, U.S.A., (# 07-081)

### Assay buffer:

*10x HEPES-Assay-Buffer pH 7.5*
*500mM HEPES*
*1mM EGTA*
*100mM DTT*
*62.5mM Na Cl*

Dissolve 770mg DTT (FLUKA cat. no. 43815; MW 154.25g/mol) [final concentration 100mM] in 30 ml of Millipore water, add 625µl of 5M NaCl₂ [final concentration 62.5mM], 19.02mg EGTA (Merck cat. No.1.06404; MW 380.35g/mol) [final concentration 1mM] and 5.96g HEPES (FLUKA cat. no. 54457) [final concentration 500mM] and adjust pH with 1M NaOH to pH 7.5. Top up to 50 ml with Millipore water. The buffer will be filtered sterile (0.22µm) aliquoted to 1.5ml and should be stored at - 20°C.

### 1M MgCl₂ :

Dissolve 10.2 g MgCl₂ x 6H₂O (FLUKA cat. no. 63068) in 50 ml of Millipore water. Buffer should be filtered (0.42µm) and can be stored at 4°C for several months.

### 1% (w/v) Pluronic F-127

Dissolve 0.5 g Pluronic (Sigma, cat. no. P-2443) in 50 ml of Millipore water. Stir gently to get a clear solution. Solution should be filtered (0.42µm) and can be stored at 4°C for several months.

### 1x HEPES-Assay-Buffer/0.1% Pluronic pH 7.5: (15ml):

*50mM HEPES*
*0.1mM EGTA*
*10mM DTT*
*0.1% Pluronic*

Add 1.5 ml 1% (w/v) Pluronic F-127 in water and 1.5 ml of 10x HEPES-Assay-Buffer pH 7.5.to 12 ml of Millipore water. Stir the mixture and check the pH value. The mixture could be stored at 4°C for 1-2 weeks.

### 0.5M EDTA (50ml):

Dissolve 9.309g EDTA (Roche Molecular Biochemicals, cat. no. 808288) in 40 ml of Millipore water and adjust pH to 8-9 with 10M NaOH in order to get a clear solution. Top up to 50 ml with Millipore water. Buffer should be filtered (0.42µm) and can be stored at 4°C for several months.

### 10mM ATP (15ml):

Dissolve 9.1mg ATP (Roche Molecular Biochemicals, cat. no. 126888) in 1.5 ml 1x HEPES-Assay-Buffer/0.1% Pluronic pH 7.5. The solution should be filtered (0.42µm) and can be stored at -20°C for 2 weeks.

### DMSO: 100% DMSO

The solvent was purchased from SIGMA (cat. No. P-2650), sterile filtered.

## Claims

1. A process for detecting acetylase or deacetylase enzyme activity, respectively, comprising the following steps:
a) providing a protein, peptide or derivative thereof comprising the sequence motif
-Z-X-Y- or -Y-X-Z-
wherein
Z = an amino acid to be acetylated or deacetylated by the respective enzyme,
X = a sequence of amino acids in the range of 0 to 10 amino acids,
Y = a discrimination enhancer for the binding to an antibody, which discrimination enhancer comprises a phosphorylated amino acid,
as a substrate for the enzyme;
b) incubating the protein, peptide, or derivative thereof with the enzyme to form a protein, peptide, or derivative thereof which is acetylated or deacetylated, respectively, at the Z position;
c) adding an antibody discriminating the modified Z position from the unmodified Z position of said protein, peptide, or derivative thereof, said discrimination being mediated by the presence of the enhancer; and
d) detecting the enzyme activity applying a homogeneous fluorescence binding immunoassay.

2. The process of claim 1 wherein the fluorescence immunoassay is a fluorescence polarization immunoassay, a fluorescence correlation spectroscopy assay, a fluorescence lifetime assay, a fluorescence resonance energy transfer assay, or a fluorescence intensity distribution assay.

3. The process of claim 1 or 2 wherein X = 1.

4. The process according to at least one of claims 1 to 3 wherein the immunoassay is performed as an homogeneous direct binding immunoassay.

5. The process according to claim 4 wherein a detectable protein, peptide or derivative thereof is used.

6. The process according to claim 4 wherein a detectable antibody is used.

7. The process according to at least one of claims 1 to 3 wherein the immunoassay is performed as an homogeneous indirect binding immunoassay.

8. The process according to claim 7 wherein a detectable ligand is added to compete with either the modified or unmodified peptide, protein or derivative thereof for binding to the antibody.

9. The process according to at least one of claims 1 to 8 wherein the process steps are performed sequentially or at least partly simultaneously.

10. A use of the assay process according to at least one of claims 1 to 9 for screening modulators of enzyme activity.

## Patentansprüche

1. Verfahren zum Nachweis von Acetylase- bzw. Deacetylase-Enzymaktivität, umfassend die folgenden Schritte:
a) Bereitstellung eines Proteins, Peptids oder Derivats davon, umfassend das Sequenzmotiv
-Z-X-Y- oder -Y-X-Z-
worin
Z = eine Aminosäure, die von dem jeweiligen Enzym acetyliert oder deacetyliert werden soll,
X = eine Sequenz von Aminosäuren im Bereich von 0 bis 10 Aminosäuren,
Y = ein Unterscheidungs-Enhancer für die Bindung an einen Antikörper, wobei der Unterscheidungs-Enhancer eine phosphorylierte Aminosäure umfasst,
als Substrat für das Enzym;
b) Inkubation des Proteins, Peptids oder Derivats davon mit dem Enzym, um ein Protein, Peptid oder Derivat davon zu bilden, welches an der Z-Position acetyliert bzw. deacetyliert ist;
c) Zugabe eines Antikörpers, welcher die modifizierte Z-Position von der unmodifizierten Z-Position des Proteins, Peptids oder Derivats davon unterscheidet, wobei die Unterscheidung durch die Anwesenheit des Enhancers vermittelt wird; und
d) Nachweis der Enzymaktivität unter Anwendung eines homogenen Fluoreszenzbindungsimmunoassays.

2. Verfahren nach Anspruch 1, wobei der Fluoreszenzimmunoassay ein Fluoreszenzpolarisationsimmunoassay, ein Fluoreszenzkorrelationsspektroskopie-Assay, ein Fluoreszenzlebensdauer-Assay, ein Fluoreszenzresonanzenergietransfer-Assay oder ein Fluoreszenzintensitätsverteilungs-Assay ist.

3. Verfahren nach Anspruch 1 oder 2, wobei X = 1.

4. Verfahren nach mindestens einem der Ansprüche 1 bis 3, wobei der Immunoassay als homogener Immunoassay mit direkter Bindung durchgeführt wird.

5. Verfahren nach Anspruch 4, wobei ein nachweisbares Protein, Peptid oder Derivat davon verwendet wird.

6. Verfahren nach Anspruch 4, wobei ein nachweisbarer Antikörper verwendet wird.

7. Verfahren nach mindestens einem der Ansprüche 1 bis 3, wobei der Immunoassay als homogener Immunoassay mit indirekter Bindung durchgeführt wird.

8. Verfahren nach Anspruch 7, wobei ein nachweisbarer Ligand zugegeben wird, um entweder mit dem modifizierten oder unmodifizierten Peptid, Protein oder Derivat davon um die Bindung an den Antikörper zu konkurrieren.

9. Verfahren nach mindestens einem der Ansprüche 1 bis 8, wobei die Verfahrensschritte sequenziell oder mindestens teilweise gleichzeitig durchgeführt werden.

10. Verwendung des Assayverfahrens nach mindestens einem der Ansprüche 1 bis 9 zum Screenen hinsichtlich Modulatoren von Enzymaktivität.

## Revendications

1. Procédé de détection de l'activité de, respectivement, l'enzyme acétylase ou de l'enzyme déacétylase, comprenant les étapes suivantes :
a) la fourniture d'une protéine, d'un peptide ou d'un dérivé de ceux-ci comprenant, en tant que substrat pour l'enzyme, le motif de séquence
-Z-X-Y- ou -Y-X-Z
dans lequel
Z = un acide aminé devant être acétylé ou déacétylé par l'enzyme correspondante,
X = une séquence d'acides aminés comportant de l'ordre de 0 à 10 acides aminés,
Y = un activateur de discrimination pour la liaison avec un anticorps, ledit activateur de discrimination comprenant un acide aminé phosphorylé ;
b) la mise en incubation de la protéine, du peptide ou du dérivé de ceux-ci avec l'enzyme, de façon à former une protéine, un peptide ou un dérivé de ceux-ci, respectivement acétylé ou déacétylé au niveau de la position Z ;
c) l'addition d'un anticorps capable de faire la différence entre la position Z modifiée et la position Z non modifiée de ladite protéine, dudit peptide ou dudit dérivé de ceux-ci, ladite discrimination étant à médiation par la présence de l'activateur ; et
d) la détection de l'activité enzymatique par application d'un dosage immunofluorescent de liaison en phase homogène.

2. Procédé selon la revendication 1, dans lequel le dosage immunofluorescent correspond à un dosage par immuno-fluoro-enzymologie, à un dosage par spectroscopie à corrélation de fluorescence, à un dosage par analyse de la durée de vie de fluorescence, à un dosage de fluorescence par transfert d'énergie de résonance (FRET) ou à un dosage par analyse de la distribution de l'intensité de fluorescence.

3. Procédé selon les revendications 1 ou 2, dans lequel X = 1.

4. Procédé selon l'une au moins des revendications 1 à 3, dans lequel le dosage immunologique est réalisé sous la forme d'un dosage immunologique par liaison directe en phase homogène.

5. Procédé selon la revendication 4, dans lequel est utilisé un peptide, une protéine ou un dérivé de ceux-ci susceptible d'être détecté.

6. Procédé selon la revendication 4, dans lequel est utilisé un anticorps susceptible d'être détecté.

7. Procédé selon l'une au moins des revendications 1 à 3, dans lequel le dosage immunologique est réalisé sous la forme d'un dosage immunologique par liaison indirecte en phase homogène.

8. Procédé selon la revendication 7, dans lequel un ligand, susceptible d'être détecté, est ajouté pour concurrencer, en matière de liaison avec l'anticorps, le peptide, la protéine ou le dérivé de ceux-ci modifié ou non modifié.

9. Procédé selon l'une au moins des revendications 1 à 8, dans lequel les étapes du procédé sont mises en oeuvre à la suite les unes des autres ou, au moins partiellement, de façon simultanée.

10. Utilisation du procédé de dosage selon au moins l'une des revendications 1 à 9 pour passer au crible des modulateurs de l'activité enzymatique.
